# EUROPEAN PATENT SPECIFICATION

(11) **EP 1 448 181 B1**
(45) Date of publication and mention of the grant of the patent: **31.05.2006**
(21) Application number: 02787576.4
(22) Date of filing: 06.11.2002
(51) Int. Cl.: A61K 31/19, A61K 31/33, A61P 3/00, A61P 9/10, A61P 25/02

(54) **PHARMACEUTICAL COMPOSITION COMPRISING A COMBINATION OF METFORMIN AND A 4-OXOBUTANOIC ACID, AND THE USE THEREOF FOR TREATING DIABETES**
PHARMAZEUTISCHE ZUBEREITUNG ENTHALTEND EINE KOMBINATION AUS METFORMIN UND A 4-OXOBUTANONSÄURE UND IHRE VERWENDUNG ZUR BEHANDLUNG VON DIABETES
COMPOSITION PHARMACEUTIQUE CONTENANT UNE COMBINAISON DE METFORMINE ET D'UN ACIDE 4-OXOBUTANOIQUE ET SON UTILISATION POUR TRAITER LE DIABETE

(30) Priority: 28.11.2001 FR 0115398
(43) Date of publication of application: 25.08.2004
(73) Proprietor: Merck Patent GmbH, 64293 Darmstadt (DE)
(72) Inventor: MOINET, Gérard, F-91400 Orsay (FR); MARAIS, Dominique, F-78250 Meulan (FR)
(86) International application number: PCT/EP2002/012355
(87) International publication number: WO 2003/045368

(56) References cited:
- WO-A-01/22951
- US-A- 5 863 915

## Description

The present invention relates to a pharmaceutical composition comprising, as active principles, metformin, optionally in the form of one of its pharmaceutically acceptable salts, and a 4-oxobutanoic acid.

The invention also relates to the use of metformin optionally in the form of one of its pharmaceutically acceptable salts and a 4-oxobutanoic acid, for the preparation of a medicinal preparation for reducing hyperglycaemia, more particularly the hyperglycaemia of non-insulin-dependent diabetes.

Diabetes is a chronic disease that has a number of pathological manifestations. It is accompanied by disorders of lipid and sugar metabolism and circulatory disorders. In many cases, diabetes tends to progress to various pathological complications. Thus, it is necessary to find a treatment that is suited to each individual suffering from diabetes.

Insulin resistance syndrome (syndrome X) is characterised by a reduction in the action of insulin (Presse Médicale, 26, No. 14, (1997), 671-677) and is involved in a great many pathological conditions, such as diabetes and more particularly non-insulin-dependent diabetes, dyslipidaemia, obesity, arterial hypertension and also certain microvascular and macrovascular complications, for instance atherosclerosis, retinopathies, nephropathies and neuropathies.

Metformin is mainly known for its anti-hyperglycaemiant activity and is widely used in the treatment of non-insulin-dependent diabetes. It improves carbohydrate homeostasis by means of its peripheral action on muscles and adipocytes by reducing the insulin resistance of diabetic patients. Thus, in the case of non-insulin-dependent diabetes, metformin is also administered to the patient in combination with insulin, since metformin is known to improve the sensitivity to insulin. Metformin also has hepatic activity, by lowering neoglucogenesis and glycogenolysis [De Fronzo, Diabetes Reviews, 6 (1998), 89-131].

Insulin secretors that allow a reduction in hyperglycaemia in the case of non-insulin-dependent diabetic patients correspond to another therapeutic category. These molecules bring about the secretion of insulin by acting on the pancreatic beta cells. They are first and foremost from the sulfonylurea family (such as gliclazide, glibenclamide and glymepiride). More recently, other types of molecules have appeared with two novel insulin secretors. These are repaglinide (a benzoic derivative) and nateglinide (a phenylalanine derivative) [H. E. Lebovitz, Diabetes Reviews, 7 (1999), 139-152].

The sulfonylurea derivatives, repaglinide and nateglinide, cause closure of the ATP-dependent potassium channel in the pancreatic beta cells. The secretion of insulin is independent of the glucose concentration [A. S. Wagman; J. M. Nuss, Current Pharmaceutical Design, 7 (2001), 417-450].

Combinations of metformin with certain insulin secretors have already been described for treating diabetes, for instance the combination metformin-glyburide, a sulfonylurea described by Bristol Myers Squibb [WO 01/32157] and the combination of repaglinide or nateglinide with an antidiabetic compound including metformin has been described by Novartis [WO 01/21159].

4-Oxobutanoic acids have already been described for treating diabetes in patent application WO 98/07681. Some of these compounds act on the early short-lived secretion of insulin.

The specific combination of metformin optionally in the form of one of its pharmaceutically acceptable salts with a 4-oxobutanoic acid has not been described, and offers particular advantages. In particular, this combination improves the diabetic patient's condition, especially by reducing the insulin resistance and affording increased control of the insulin resistance in response to glucose.

Thus, one aim of the present invention is to propose a composition for significantly improving the diabetic patient's condition.

An aim of the invention is also to propose a composition that is suited to the treatment of diabetes by means of a conjugate effect on insulin resistance syndrome and on the early short-lived secretion of insulin.

Finally, an aim of the invention is to propose a composition that is particularly suitable for reducing hyperglycaemia and more particularly the hyperglycaemia of non-insulin-dependent diabetes.

These aims and others are achieved by the present invention, which relates to a pharmaceutical composition comprising, as active principles, metformin, optionally in the form of one of its pharmaceutically acceptable salts, and a compound of the formula (I), in combination with one or more pharmaceutically acceptable excipients.

This composition is particularly suitable for treating diabetes, more particularly non-insulin-dependent diabetes. It is particularly suitable for reducing the hyperglycaemia of non-insulin-dependent diabetes.

It is also suitable for treating pathologies associated with insulin resistance syndrome, such as, especially, dyslipidaemia, obesity, arterial hypertension, and microvascular and macrovascular complications, for instance atherosclerosis, retinopathies, nephropathies and neuropathies.

The compound of the formula (I) is defined as follows: 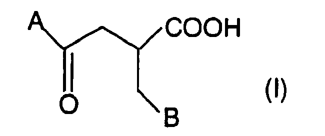 in which the groups A and B are chosen, independently of each other, from:
- a mono-, bi- or tricyclic aryl group containing from 6 to 14 carbon atoms;
- a heteroaromatic group chosen from pyridyl, pyrimidyl, pyrrolyl, furyl and thienyl groups;
- an alkyl group containing from 1 to 14 carbon atoms;
- a cycloalkyl group containing from 5 to 8 carbon atoms;
- a saturated heterocyclic group chosen from tetrahydrofuryl, tetrahydropyranyl, piperidyl and pyrrolidinyl groups;
the groups A and B possibly bearing 1 to 3 substituents chosen from a C₁-C₆ alkyl group, a C₁-C₆ alkoxy group, a C₆-C₁₄ aryl group, a heteroaryl group chosen from pyridyl, pyrimidyl, pyrrolyl, furyl and thienyl, a (C₆-C₁₄)aryl(C₁-C₆)-alkyl group, a (C₆-C₁₄)aryl(C₁-C₆)alkyl(C₆-C₁₄)aryl group, a halogen or a trifluoromethyl, trifluoromethoxy, cyano, hydroxyl, nitro, amino, carboxyl, (C₁-C₆)alkoxycarbonyl, carbamoyl, (C₁-C₆)alkylsulfonyl, sulfoamino, (C₁-C₆)alkylsulfonylamino, sulfamoyl or (C₁-C₆)alkylcarbonylamino group;
or two of the substituents forming a methylenedioxy group, a solvate thereof or a salt of this acid.

In a preferred embodiment of the invention, the 4-oxobutanoic acids are those of the formula (II) in which A and B are chosen from aryl groups.

Examples of aryl groups that may be mentioned include phenyl, α-naphthyl, β-naphthyl and fluorenyl groups.

The C₁-C₆ alkyl groups may be linear or branched. Examples that may be mentioned include methyl, ethyl, propyl, isopropyl, butyl, isobutyl, tert-butyl and pentyl groups.

The C₁-C₆ alkoxy groups may also be linear or branched.

Examples that may be mentioned include methoxy, ethoxy, propoxy, isopropoxy, butoxy and isobutoxy groups.

The halogens may be chosen from fluorine, chlorine, bromine and iodine.

The present invention also includes the tautomeric forms of the compounds of the general formula (I), the enantiomers, diastereoisomers and epimers of these compounds, and also the solvates thereof.

Examples of salts of the compounds of the general formula (I) include pharmacologically acceptable salts, such as the sodium salts, potassium salts, magnesium salts, calcium salts, amine salts and other salts of the same type (aluminium, iron, bismuth, etc.).

In a preferred embodiment, the 4-oxobutanoic acids are chosen from:
- 2-benzyl-4-(4-methoxyphenyl)-4-oxobutanoic acid
- 2-benzyl-4-(4-fluorophenyl)-4-oxobutanoic acid
- 2-cyclohexylmethyl-4-(4-methoxyphenyl)-4-oxobutanoic acid
- 2-benzyl-4-phenyl-4-oxobutanoic acid
- 2-(β-naphthylmethyl)-4-phenyl-4-oxobutanoic acid
- 2-benzyl-4-(β-naphthyl)-4-oxobutanoic acid
- 2-[(4-chlorophenyl)methyl]-4-(4-methoxyphenyl)-4-oxobutanoic acid
- 2-benzyl-4-(4-methylphenyl)-4-oxobutanoic acid
- 4-(4-fluorophenyl)-2-[(4-methoxyphenyl)methyl]-4-oxobutanoic acid
- 2-benzyl-4-(3,4-methylenedioxyphenyl)-4-oxobutanoic acid
- 2-benzyl-4-cyclohexyl-4-oxobutanoic acid
- 4-phenyl-2-[(tetrahydrofur-2-yl)methyl]-4-oxobutanoic acid,
the solvates, enantiomers and salts of these acids.

The 4-oxobutanoic acid is advantageously chosen from:
- (-)-2-benzyl-4-(4-methoxyphenyl)-4-oxobutanoic acid
- (+)-2-benzyl-4-(4-methoxyphenyl)-4-oxobutanoic acid
- (-)-2-benzyl-4-(4-fluorophenyl)-4-oxobutanoic acid
- (+)-2-benzyl-4-(4-fluorophenyl)-4-oxobutanoic acid.

According to the invention, metformin or 1,1-dimethylbiguanide may be administered in the form of one of its pharmaceutically acceptable salts, such as the hydrochloride, acetate, benzoate, citrate, fumarate, embonate, chlorophenoxyacetate, glycolate, palmoate, aspartate, methanesulfonate, maleate, para-chlorophenoxyisobutyrate, formate, lactate, succinate, sulfate, tartrate, cyclohexanecarboxylate, hexanoate, octanoate, decanoate, hexadecanoate, octadecanoate, benzenesulfonate, trimethoxybenzoate, para-toluenesulfonate, adamantanecarboxylate, glyoxylate, glutamate, pyrrolidonecarboxylate, naphthalenesulfonate, glucose-1-phosphate, nitrate, sulfite, dithionate, phosphate, dobesilate, thioctate, hippurate, 3-benzamidopropanoate, glucuronate, L-pyrrolidone-5-carboxylate, cholate, α-glucose-1-phosphate, alginate or 4-aminobenzoate, and the salt with chondroitinsulfuric acid.

Among these salts, the hydrochloride, fumarate, embonate and chlorophenoxyacetate are more particularly preferred.

The pharmaceutically acceptable salts of metformin are obtained, in a manner that is known per se, by the action of metformin on the corresponding acid.

The compositions of the invention comprise therapeutically effective amounts of the various active principles. The ratios of the respective amounts of metformin and of compound of the formula (I) thus vary in consequence.

The weight ratio of metformin or of its pharmaceutically acceptable salt to the compound of the formula (I) preferably ranges from 1/1, preferably from 40/1 and better still from 2/1, to 20/1.

The compositions of the invention are preferably administered parenterally or better still orally, although the other routes of administration, such as, for example, rectal administration, are not excluded.

If oral administration is envisaged, the compositions of the invention are in the form of gel capsules, effervescent tablets, coated or uncoated tablets, sachets, sugar-coated tablets, drinkable vials or solutions, microgranules or sustained-release forms.

If parenteral administration is envisaged, the compositions of the invention are in the form of injectable solutions and suspensions packaged in vials or bottles for slow venous infusion.

The forms for oral administration are prepared by mixing the active substance with various types of excipients or of vehicles, such as fillers, disintegration (or crumbling) agents, binders, colorants, flavour enhancers and the like, followed by shaping of the mixture.

The colorant can be any colorant permitted for pharmaceutical use.

Examples of flavour enhancers include cocoa powder, mint, borneol and cinnamon powder.

Examples of binders that may be mentioned are polyvinylpyrrolidone, hydroxypropylmethylcellulose, alginic acid, carbomer, carboxymethylcellulose, dextrin, ethylcellulose, starch, sodium alginate, polymethacrylate, maltodextrin, liquid glucose, magnesium aluminium silicate, hydroxyethylcellulose, ethylcellulose, methylcellulose and guar gum.

It is possible to use alginic acid, sodium carboxymethylcellulose, colloidal silicon dioxide, sodium croscarmellose, crospovidone, guar gum, magnesium aluminium silicate, methylcellulose, microcrystalline cellulose, potassium polacrilin, cellulose powder, pre-gelatinised starch, sodium alginate or sodium starch glycolate as disintegration agent.

The fillers are, for example, cellulose, lactose, calcium hydrogen phosphate or microcrystalline cellulose.

The tablets can be obtained in a conventional manner by compressing granules in the presence of one or more lubricants. Suitable lubricants are calcium stearate, glyceryl monostearate, glyceryl palmitostearate, hydrogenated castor oil, hydrogenated plant oil, light mineral oil, magnesium stearate, polyethylene glycol, sodium benzoate, sodium lauryl sulfate, stearyl sodium fumarate, stearic acid, talc and zinc stearate. These tablets can then be coated using polymers in solution or suspension, such as hydroxypropylmethylcellulose or ethylcellulose.

The granules used to do this are prepared, for example, by using the wet granulation process starting with a mixture of the active principles with one or more excipients, such as a binder, a crumbling agent (or disintegration agent) and a filler.

To obtain hard capsules, the mixture of active principles with a suitable filler (for example lactose) is incorporated into empty gelatin capsules optionally in the presence of a lubricant, such as magnesium stearate, stearic acid, talc or zinc stearate.

Gel capsules or soft capsules are prepared by dissolving the active principles in a suitable solvent (for example polyethylene glycol), followed by incorporation into soft capsules.

The forms for parenteral administration are obtained in a conventional manner by mixing the active principles with buffers, stabilisers, preserving agents, solubilising agents, tonicity agents and suspension agents. In accordance with the known techniques, these mixtures are subsequently sterilised and then packaged in the form of intravenous injections.

As buffer, a person skilled in the art can use buffers based on organophosphate salts.

Examples of suspension agents include methylcellulose, hydroxyethylcellulose, acacia and sodium carboxymethylcellulose.

Examples of solubilising agents include castor oil solidified with polyoxyethylene, polysorbate 80, nicotinamide and macrogol.

In addition, stabilisers that are useful according to the invention are sodium sulfite and sodium metasulfite, while mention may be made of sodium p-hydroxybenzoate, sorbic acid, cresol and chlorocresol as preserving agents. For the preparation of an oral solution or suspension, the active principles are dissolved or suspended in a suitable vehicle with a dispersant, a wetting agent, a suspension agent (for example polyvinylpyrrolidone), a preserving agent (such as methylparaben or propylparaben), a flavour enhancer or a colorant.

For the preparation of suppositories, the active principles are mixed in a manner that is known per se with a suitable base constituent, such as polyethylene glycol or semisynthetic glycerides.

For the preparation of microcapsules, the active principles are combined with suitable diluents, suitable stabilisers, agents that promote the sustained release of the active substances or any other type of additive for the formation of a central core that is then coated with a suitable polymer (for example a water-soluble resin or a water-insoluble resin). The techniques known to those skilled in the art will be used for this purpose.

The microcapsules thus obtained are then optionally formulated in suitable dosage units.

The present invention also relates to the use of metformin, optionally in the form of one of its pharmaceutical acceptable salts, in combination with a compound of the formula (I) as defined above for the preparation of a medicinal combination for treating diabetes, more particularly non-insulin-dependent diabetes.

The treatment of diabetes, more particularly non-insulin-dependent diabetes, in a mammal, comprises the administration to the said mammal of the composition according to the present invention.

The metformin may be in the form of any of the salts defined above; however, it is preferred to use metformin in unmodified form or in the form of the hydrochloride, fumarate, embonate or chlorophenoxyacetate.

If metformin or its salt and the compound of the formula (I) are incorporated into the same unit dose, the unit dose preferably comprises from 50 to 1000 mg of metformin.

In this case, the unit dose advantageously comprises from 12.5 to 400 mg of compound of the formula (I).

Naturally, the dosage depends on the method of administration, the therapeutic indication, and the age and condition of the patient.

In general, the daily dosage ranges between 100 and 2000 mg of metformin and between 12.5 and 400 mg of compound of the formula (I).

Specific but non-limiting examples of the invention will now be presented.

### EXAMPLE 1

A tablet having the composition below is prepared:

| | mass in mg | weight % |
|---|---|---|
| Compound P | 50 | 7.7 |
| Metformin | 500 | 76.7 |
| Microcrystalline cellulose | 40 | 6.1 |
| Fine lactose powder | 30 | 4.6 |
| Hydroxypropylcellulose | 12 | 1.8 |
| Sodium croscarmellose | 12 | 1.8 |
| Colloidal silica (Aerosil®) | 2 | 0.3 |
| Magnesium stearate | 6 | 0.9 |

| | | |
|---|---|---|
| Compound P: (+)-2-benzyl-4-(4-fluorophenyl)-4-oxobutanoic acid. | | |

### EXAMPLE 2

A tablet having the composition below is prepared:

| | mass in mg | weight % |
|---|---|---|
| Compound P | 50 | 4.8 |
| Metformin | 850 | 81.1 |
| Microcrystalline cellulose | 50 | 4.8 |
| Fine lactose powder | 45 | 4.3 |
| Hydroxypropylcellulose | 20 | 1.9 |
| Sodium croscarmellose | 20 | 3.1 |
| Colloidal silica (Aerosil®) | 3 | 0.5 |
| Magnesium stearate | 10 | 1.0 |

| | | |
|---|---|---|
| Compound P: (+)-2-benzyl-4-(4-fluorophenyl)-4-oxobutanoic acid. | | |

### EXAMPLE 3

A tablet having the composition below is prepared:

| | mass in mg | weight % |
|---|---|---|
| Compound P | 100 | 14.0 |
| Metformin | 500 | 69.9 |
| Microcrystalline cellulose | 40 | 5.6 |
| Fine lactose powder | 36 | 5.0 |
| Hydroxypropylcellulose | 15 | 2.1 |
| Sodium croscarmellose | 15 | 2.3 |
| Colloidal silica (Aerosil®) | 2 | 0.3 |
| Magnesium stearate | 7 | 1.0 |

| | | |
|---|---|---|
| Compound P: (+)-2-benzyl-4-(4-fluorophenyl)-4-oxobutanoic acid. | | |

### EXAMPLE 4

A tablet having the composition below is prepared:

| | mass in mg | weight % |
|---|---|---|
| Compound P | 100 | 9.1 |
| Metformin | 850 | 77.4 |
| Microcrystalline cellulose | 50 | 4.6 |
| Fine lactose powder | 44 | 4.0 |
| Hydroxypropylcellulose | 20 | 1.8 |
| Sodium croscarmellose | 21 | 3.2 |
| Colloidal silica (Aerosil®) | 3 | 0.5 |
| Magnesium stearate | 10 | 0.9 |

| | | |
|---|---|---|
| Compound P: (+)-2-benzyl-4-(4-fluorophenyl)-4-oxobutanoic acid. | | |

### EXAMPLE 5

A tablet having the composition below is prepared:

| | mass in mg | weight % |
|---|---|---|
| Compound P | 200 | 23.8 |
| Metformin | 500 | 59.6 |
| Microcrystalline cellulose | 50 | 6.0 |
| Fine lactose powder | 42 | 5.0 |
| Hydroxypropylcellulose | 18 | 2.1 |
| Sodium croscarmellose | 20 | 3.1 |
| Colloidal silica (Aerosil®) | 2 | 0.3 |
| Magnesium stearate | 7 | 0.8 |

| | | |
|---|---|---|
| Compound P: (+)-2-benzyl-4-(4-fluorophenyl)-4-oxobutanoic acid. | | |

### EXAMPLE 6

A tablet having the composition below is prepared:

| | mass in mg | weight % |
|---|---|---|
| Compound P | 200 | 16.6 |
| Metformin | 850 | 70.4 |
| Microcrystalline cellulose | 50 | 4.1 |
| Fine lactose powder | 45 | 3.7 |
| Hydroxypropylcellulose | 24 | 2.0 |
| Sodium croscarmellose | 25 | 3.8 |
| Colloidal silica (Aerosil®) | 3 | 0.5 |
| Magnesium stearate | 10 | 0.8 |

| | | |
|---|---|---|
| Compound P: (+)-2-benzyl-4-(4-fluorophenyl)-4-oxobutanoic acid. | | |

## Claims

1. Pharmaceutical composition comprising, as active principles, (i) metformin, optionally in the form of one of its pharmaceutically acceptable salts, and (ii) a compound of the formula (I), in combination with one or more pharmaceutically acceptable excipients, the compound of the formula (I) being defined as follows: 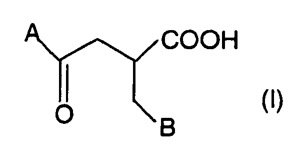 in which the groups A and B are chosen, independently of each other, from:
- a mono-, bi- or tricyclic aryl group containing from 6 to 14 carbon atoms;
- a heteroaromatic group chosen from pyridyl, pyrimidyl, pyrrolyl, furyl and thienyl groups;
- an alkyl group containing from 1 to 14 carbon atoms;
- a cycloalkyl group containing from 5 to 8 carbon atoms;
- a saturated heterocyclic group chosen from tetrahydrofuryl, tetrahydropyranyl, piperidyl and pyrrolidinyl groups;
the groups A and B possibly bearing 1 to 3 substituents chosen from a C₁-C₆ alkyl group, a C₁-C₆ alkoxy group, a C₆-C₁₄ aryl group, a heteroaryl group chosen from pyridyl, pyrimidyl, pyrrolyl, furyl and thienyl, a (C₆-C₁₄)aryl(C₁-C₆)-alkyl group, a (C₆-C₁₄)aryl(C₁-C₆)alkyl(C₆-C₁₄)aryl group, a halogen or a trifluoromethyl, trifluoromethoxy, cyano, hydroxyl, nitro, amino, carboxyl, (C₁-C₆)alkoxycarbonyl, carbamoyl, (C₁-C₆)alkylsulfonyl, sulfoamino, (C₁-C₆)alkylsulfonylamino, sulfamoyl or (C₁-C₆)alkylcarbonylamino group;
or two of the substituents forming a methylenedioxy group, a solvate thereof or a salt of this acid.

2. Composition according to Claim 1, **characterised in that** the 4-oxobutanoic acid is of the formula (I) in which A and B are chosen from aryl groups.

3. Composition according to Claim 1 or 2, for treating diabetes.

4. Composition according to any one of Claims 1 to 3, for treating non-insulin-dependent diabetes.

5. Composition according to Claim 1 or 2, for treating at least one of the pathologies associated with insulin resistance syndrome, more particularly chosen from dyslipidaemia, obesity, arterial hypertension, and microvascular and macrovascular complications, for instance atherosclerosis, retinopathies, nephropathies and neuropathies.

6. Pharmaceutical composition according to any one of Claims 1 to 5, **characterised in that** the weight ratio of metformin or of its pharmaceutically acceptable salt to the compound of the formula (I) ranges from 1/1 to 40/1.

7. Pharmaceutical composition according to any one of the preceding claims, **characterised in that** the metformin salt is a hydrochloride, fumarate, embonate or chlorophenoxyacetate.

8. Composition according to any one of the preceding claims, **characterised in that** the compound of the formula (I) is chosen from:
- 2-benzyl-4-(4-methoxyphenyl)-4-oxobutanoic acid
- 2-benzyl-4-(4-fluorophenyl)-4-oxobutanoic acid
- 2-cyclohexylmethyl-4-(4-methoxyphenyl)-4-oxobutanoic acid
- 2-benzyl-4-phenyl-4-oxobutanoic acid
- 2-(β-naphthylmethyl)-4-phenyl-4-oxobutanoic acid
- 2-benzyl-4-(β-naphthyl)-4-oxobutanoic acid
- 2-[(4-chlorophenyl)methyl]-4-(4-methoxyphenyl)-4-oxobutanoic acid
- 2-benzyl-4-(4-methylphenyl)-4-oxobutanoic acid
- 4-(4-fluorophenyl)-2-[(4-methoxyphenyl)methyl]-4-oxobutanoic acid
- 2-benzyl-4-(3,4-methylenedioxyphenyl)-4-oxobutanoic acid
- 2-benzyl-4-cyclohexyl-4-oxobutanoic acid
- 4-phenyl-2-[(tetrahydrofur-2-yl)methyl]-4-oxobutanoic acid, the solvates, enantiomers and salts of these acids.

9. Composition according to Claim 8, **characterised in that** the compound of the formula (I) is chosen from:
- (-)-2-benzyl-4-(4-methoxyphenyl)-4-oxobutanoic acid
- (+)-2-benzyl-4-(4-methoxyphenyl)-4-oxobutanoic acid
- (-)-2-benzyl-4-(4-fluorophenyl)-4-oxobutanoic acid
- (+)-2-benzyl-4-(4-fluorophenyl)-4-oxobutanoic acid.

10. Composition according to any one of the preceding claims, that is suitable for oral administration.

11. Use of metformin, optionally in the form of one of its pharmaceutically acceptable salts, in combination with a compound of the formula (I) as defined in Claim 1 for the preparation of a medicinal combination for treating diabetes.

12. Use of metformin, optionally in the form of one of its pharmaceutically acceptable salts, in combination with a compound of the formula (I) as defined in Claim 1 for the preparation of a medicinal combination for treating non-insulin-dependent diabetes.

13. Use of metformin, optionally in the form of one of its pharmaceutically acceptable salts, in combination with a compound of the formula (I) as defined in Claim 1 for the preparation of a medicinal combination for treating at least one of the pathologies associated with insulin resistance syndrome, more particularly chosen from dyslipidaemia, obesity, arterial hypertension, and microvascular and macrovascular complications, for instance atherosclerosis, retinopathies, nephropathies and neuropathies.

14. Use according to any one of Claims 11 to 13, **characterised in that** the metformin salt is a hydrochloride, a fumarate, an embonate or a chlorophenoxyacetate.

15. Use according to one of Claims 11 to 14, **characterised in that** the compound of the formula (I) is chosen from:
- 2-benzyl-4-(4-methoxyphenyl)-4-oxobutanoic acid
- 2-benzyl-4-(4-fluorophenyl)-4-oxobutanoic acid
- 2-cyclohexylmethyl-4-(4-methoxyphenyl)-4-oxobutanoic acid
- 2-benzyl-4-phenyl-4-oxobutanoic acid
- 2-(β-naphthylmethyl)-4-phenyl-4-oxobutanoic acid
- 2-benzyl-4-(β-naphthyl)-4-oxobutanoic acid
- 2-[(4-chlorophenyl)methyl]-4-(4-methoxyphenyl)-4-oxobutanoic acid
- 2-benzyl-4-(4-methylphenyl)-4-oxobutanoic acid
- 4-(4-fluorophenyl)-2-[(4-methoxyphenyl)methyl]-4-oxobutanoic acid
- 2-benzyl-4-(3,4-methylenedioxyphenyl)-4-oxobutanoic acid
- 2-benzyl-4-cyclohexyl-4-oxobutanoic acid
- 4-phenyl-2-[(tetrahydrofur-2-yl)methyl]-4-oxobutanoic acid,
the solvates, enantiomers and salts of these acids.

16. Use according to one of Claims 11 to 14, **characterised in that** the compound of the formula (I) is chosen from:
- (-)-2-benzyl-4-(4-methoxyphenyl)-4-oxobutanoic acid
- (+)-2-benzyl-4-(4-methoxyphenyl)-4-oxobutanoic acid
- (-)-2-benzyl-4-(4-fluorophenyl)-4-oxobutanoic acid
- (+)-2-benzyl-4-(4-fluorophenyl)-4-oxobutanoic acid.

17. Use according to any one of Claims 11 to 16, **characterised in that** the medicinal combination is in the form of a unit dose comprising metformin or one of its pharmaceutically acceptable salts, and a compound of the formula (I).

18. Use according to Claim 17, **characterised in that** the unit dose comprises from 50 to 1000 mg of metformin and from 12.5 to 400 mg of compound of the formula (I).

## Patentansprüche

1. Pharmazeutische Zusammensetzung, enthaltend als Wirkstoffe (i) Metformin, gegebenenfalls in Form eines seiner pharmazeutisch unbedenklichen Salze, und (ii) eine Verbindung der Formel (I) in Kombination mit einem oder mehreren pharmazeutisch unbedenklichen Träger, wobei die Verbindung der Formel (I) wie folgt definiert ist: 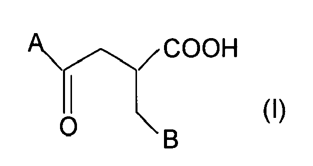 worin die Gruppen A und B unabhängig voneinander ausgewählt sind aus:
- einer mono-, bi- oder tricyclischen Arylgruppe mit 6 bis 14 Kohlenstoffatomen;
- einer heteroaromatischen Gruppe, die aus Pyridyl-, Pyrimidyl-, Pyrrolyl-, Furyl- und Thienylgruppen ausgewählt ist;
- einer Alkylgruppe mit 1 bis 14 Kohlenstoffatomen;
- einer Cycloalkylgruppe mit 5 bis 8 Kohlenstoffatomen;
- einer gesättigten heterocyclischen Gruppe, die aus Tetrahydrofuryl-, Tetrahydropyranyl-, Piperidyl- und Pyrrolidinylgruppen ausgewählt ist;
wobei die Gruppen A und B gegebenenfalls 1 bis 3 Substituenten tragen, die aus einer C₁-C₆-Alkylgruppe, einer C₁-C₆-Alkoxygruppe, einer C₆-C₁₄₋Arylgruppe, einer Heteroarylgruppe, die aus Pyridyl-, Pyrimidyl-, Pyrrolyl-, Furyl- und Thienyl ausgewählt ist, einer (C₆-C₁₄)Aryl(C₁-C₆)alkylgruppe, einer (C₆-C₁₄)-Aryl(C₁-C₆)alkyl(C₆-C₁₄)arylgruppe, einem Halogen oder einer Trifluor methyl-, Trifluormethoxy-, Cyano-, Hydroxyl-, Nitro-, Amino-, Carboxyl-, (C₁-C₆)Alkoxycarbonyl-, Carbamoyl-, (C₁-C₆)Alkylsulfonyl, Sulfoamino-, (C₁-C₆)-Alkylsulfonyl-amino-, Sulfamoyl- oder (C₁-C₆)Alkylcarbonylaminogruppe ausgewählt sind;
oder wobei zwei der Substituenten eine Methylendioxygruppe bilden, ein Solvat davon oder ein Salz dieser Säure.

2. Zusammensetzung nach Anspruch 1, **dadurch gekennzeichnet, dass** 4-Oxobutansäure die Formel (I) hat, in der A und B aus Arylgruppen ausgewählt sind.

3. Zusammensetzung nach Anspruch 1 oder 2 zur Behandlung von Diabetes.

4. Zusammensetzung nach einem der Ansprüche 1 bis 3 zur Behandlung von nicht-insulinabhängigem Diabetes.

5. Zusammensetzung nach Anspruch 1 oder 2 zur Behandlung mindestens einer der Pathologien, die mit dem Insulinresistenzsyndrom einhergehen, insbesondere ausgewählt aus Dyslipidämie, Fettsucht, arterieller Hypertonie und mikrovaskulären und makrovaskulären Komplikationen, zum Beispiel Atherosklerose, Retinopathien, Nephropathien und Neuropathien.

6. Pharmazeutische Zusammensetzung nach einem der Ansprüche 1 bis 5, **dadurch gekennzeichnet, dass** das Gewichtsverhältnis von Metformin oder seinem pharmazeutisch unbedenklichen Salz zu der Verbindung der Formel (I) im Bereich von 1/1 bis 40/1 liegt.

7. Pharmazeutische Zusammensetzung nach einem der vorhergehenden Ansprüche, **dadurch gekennzeichnet, dass** das Metforminsalz ein Hydrochlorid, Fumarat, Embonat oder Chlorphenoxyacetat ist.

8. Zusammensetzung nach einem der vorhergehenden Ansprüche, **dadurch gekennzeichnet, dass** die Verbindung der Formel (I) ausgewählt ist aus:
- 2-Benzyl-4-(4-methoxyphenyl)-4-oxobutansäure
- 2-Benzyl-4-(4-fluorphenyl)-4-oxobutansäure
- 2-Cyclohexylmethyl-4-(4-methoxyphenyl)-4-oxobutansäure
- 2-Benzyl-4-phenyl-4-oxobutansäure
- 2-(β-Naphthylmethyl)-4-phenyl-4-oxobutansäure
- 2-Benzyl-4-(β-naphthyl)-4-oxobutansäure
- 2-[(4-Chlorphenyl)methyl]-4-(4-methoxyphenyl)-4-oxobutansäure
- 2-Benzyl-4-(4-methylphenyl)-4-oxobutansäure
- 4-(4-Fluorphenyl)-2-[(4-methoxyphenyl)methyl]-4-oxobutansäure
- 2-Benzyl-4-(3,4-methylendioxyphenyl)-4-oxobutansäure
- 2-Benzyl-4-cyclohexyl-4-oxobutansäure
- 4-Phenyl-2-[(tetrahydrofur-2-yl)methyl]-4-oxobutansäure,
den Solvaten, Enantiomeren und Salzen dieser Säuren.

9. Zusammensetzung nach Anspruch 8, **dadurch gekennzeichnet, dass** die Verbindung der Formel (I) ausgewählt ist aus:
- (-)-2-Benzyl-4-(4-methoxyphenyl)-4-oxobutansäure
- (+)-2-Benzyl-4-(4-methoxyphenyl)-4-oxobutansäure
- (-)-2-Benzyl-4-(4-fluorphenyl)-4-oxobutansäure
- (+)-2-Benzyl-4-(4-fluorphenyl)-4-oxobutansäure.

10. Zusammensetzung nach einem der vorhergehenden Ansprüche, die sich zur oralen Verabreichung eignet.

11. Verwendung von Metformin, gegebenenfalls in Form eines seiner pharmazeutisch unbedenklichen Salze, in Kombination mit einer Verbindung der Formel (I) nach Anspruch 1 zur Herstellung einer medizinischen Kombination zur Behandlung von Diabetes.

12. Verwendung von Metformin, gegebenenfalls in Form eines seiner pharmazeutisch unbedenklichen Salze, in Kombination mit einer Verbindung der Formel (I) nach Anspruch 1 zur Herstellung einer medizinischen Kombination zur Behandlung von nicht-insulinabhängigem Diabetes.

13. Verwendung von Metformin, gegebenenfalls in Form eines seiner pharmazeutisch unbedenklichen Salze, in Kombination mit einer Verbindung der Formel (I) nach Anspruch 1 zur Herstellung einer medizinischen Kombination zur Behandlung von mindestens einer der Pathologien, die mit dem Insulinresistenzsyndrom einhergehen, insbesondere ausgewählt aus Dyslipidämie, Fettsucht, arterieller Hypertonie und mikrovaskulären und makrovaskulären Komplikationen, zum Beispiel Atherosklerose, Retinopathien, Nephropathien und Neuropathien.

14. Verwendung nach einem der Ansprüche 11 bis 13, **dadurch gekennzeichnet, dass** das Metforminsalz ein Hydrochlorid, ein Fumarat, ein Embonat oder ein Chlorphenoxyacetat ist.

15. Verwendung nach einem der Ansprüche 11 bis 14, **dadurch gekennzeichnet, dass** die Verbindung der Formel (I) ausgewählt ist aus:
- 2-Benzyl-4-(4-methoxyphenyl)-4-oxobutansäure
- 2-Benzyl-4-(4-fluorphenyl)-4-oxobutansäure
- 2-Cyclohexylmethyl-4-(4-methoxyphenyl)-4-oxobutansäure
- 2-Benzyl-4-phenyl-4-oxobutansäure
- 2-(β-Naphthylmethyl)-4-phenyl-4-oxobutansäure
- 2-Benzyl-4-(β-naphthyl)-4-oxobutansäure
- 2-[(4-Chlorphenyl)methyl]-4-(4-methoxyphenyl)-4-oxobutansäure
- 2-Benzyl-4-(4-methylphenyl)-4-oxobutansäure
- 4-(4-Fluorphenyl)-2-[(4-methoxyphenyl)methyl]-4-oxobutansäure
- 2-Benzyl-4-(3,4-methylendioxyphenyl)-4-oxobutansäure
- 2-Benzyl-4-cyclohexyl-4-oxobutansäure
- 4-Phenyl-2-[(tetrahydrofur-2-yl)methyl]-4-oxobutansäure,
den Solvaten, Enantiomeren und Salzen dieser Säuren.

16. Verwendung nach einem der Ansprüche 11 to 14, **dadurch gekennzeichnet, dass** die Verbindung der Formel (I) ausgewählt ist aus:
- (-)-2-Benzyl-4-(4-methoxyphenyl)-4-oxobutansäure
- (+)-2-Benzyl-4-(4-methoxyphenyl)-4-oxobutansäure
- (-)-2-Benzyl-4-(4-fluorphenyl)-4-oxobutansäure
- (+)-2-Benzyl-4-(4-fluorphenyl)-4-oxobutansäure.

17. Verwendung nach einem der Ansprüche 11 to 16, **dadurch gekennzeichnet, dass** die medizinische Kombination die Form einer Einheitsdosis hat, die Metformin oder eines seiner pharmazeutisch unbedenklichen Salze und eine Verbindung der Formel (I) enthält.

18. Verwendung nach Anspruch 17, **dadurch gekennzeichnet, dass** die Einheitsdosis 50 bis 1000 mg Metformin und 12,5 bis 400 mg der Verbindung der Formel (I) enthält.

## Revendications

1. Composition pharmaceutique comprenant, en tant que principes actifs, (i) de la metformine éventuellement sous la forme de l'un de ses sels pharmaceutiquement acceptables et (ii) un composé de formule (I) en association avec un ou plusieurs excipients pharmaceutiquement acceptables, le composé de formule (I) étant défini de la manière suivante : 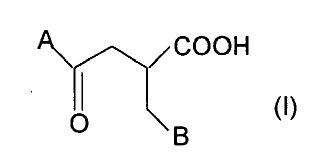 dans laquelle les groupes A et B sont choisis indépendamment l'un de l'autre parmi :
- un groupe aryle mono-, bi- ou tricyclique ayant de 6 à 14 atomes de carbone ;
- un groupe hétéroaromatique choisi parmi les groupes pyridyle, pyrimidyle, pyrrolyle, furyle et thiényle ;
- un groupe alcoyle ayant de 1 à 14 atomes de carbone ;
- un groupe cycloalcoyle ayant de 5 à 8 atomes de carbone ;
- un groupe hétérocyclique saturé choisi parmi les groupes tétra-hydrofuryle, tétrahydropyrranyle, pipéridinyle et pyrrolidinyle ;
les groupes A et B pouvant porter 1 à 3 substituants choisis parmi un groupe alcoyle en C₁-C₆, alcoxy en C₁-C₆, aryle en C₆-C₁₄, hétéroaryle choisi parmi pyridyle, pyrimidyle, pyrrolyle, furyle et thiényle, aryl (C₆-C₁₄) alcoyle (C₁-C₆), aryl (C₆-C₁₄) alcoyl (C₁-C₆) aryle (C₆-C₁₄), un halogène, un groupe trifluorométhyle, trifluorométhoxy, cyano, hydroxy, nitro, amino, carboxy, alcoxy (C₁-C₆) carbonyle, carbamoyle, alcoyl(C₁-C₆)sulfonyle, sulfoamino, alcoyl(C₁-C₆) sulfonylamino, sulfamoyle, alcoyl(C₁-C₆)carbonylamino
ou deux des substituants formant un groupe methylènedioxy, son solvate ou un sel de cet acide.

2. Composition selon la revendication 1, **caractérisée en ce que** l'acide 4-oxo-butanoïque est de formule (I) dans laquelle A et B sont choisis parmi des groupes aryle.

3. Composition selon la revendication 1 ou 2, pour traiter le diabète.

4. Composition selon l'une quelconque des revendications 1 à 3, pour traiter le diabète non insulino-dépendant.

5. Composition selon la revendication 1 ou 2, pour traiter au moins une des pathologies associées au syndrome d'insulinorésistance, plus particulièrement choisie parmi la dyslipidémie, l'obésité, l'hypertension artérielle, les complications micro- et macro-vasculaires, comme l'athérosclérose, les rétinopathies, les néphropathies et les neuropathies.

6. Composition pharmaceutique selon l'une quelconque des revendications 1 à 5, **caractérisée en ce que** le rapport pondéral de la metformine ou de son sel pharmaceutiquement acceptable au composé de formule (I) varie de 1/1 à 40/1.

7. Composition pharmaceutique selon l'une quelconque des revendications précédentes, **caractérisée en ce que** le sel de metformine est un chlorhydrate, fumarate, embonate ou chlorophénoxyacétate.

8. Composition selon l'une quelconque des revendications précédentes, **caractérisée en ce que** le composé de formule (I) est choisi parmi :
l'acide 2-benzyl-4-(4-méthoxyphényl)-4-oxobutanoïque
l'acide 2-benzyl-4-(4-fluorophényl)-4-oxobutanoïque
l'acide 2-cyclohexylméthyl-4-(4-méthoxyphényl)-4-oxobutanoïque
l'acide 2-benzyl-4-phényl-4-oxobutanoïque
l'acide 2-(β-nap hthylméthyl)-4-phényl-4-oxobutanoïque
l'acide 2-benzyl-4-(β-naphtyl)-4-oxobutanoïque
l'acide 2-[(4-chlorophényl)méthyl]-4-(4-méthoxyphényl)-4-oxobutanoïque
l'acide 2-benzyl-4-(4-méthylphényl)-4-oxobutanoïque
l'acide 4-(4-fluorophényl)-2-[(4-méthoxyphényl)méthyl]-4-oxobutanoïque
l'acide 2-benzyl-4-(3,4-méthylènedioxyphényl)-4-oxobutanoïque
l'acide 2-benzyl-4-cyclohexyl-4-oxobutanoïque
l'acide 4-phényl-2-[(tétrahydrofur-2-yl)méthyl)-4-oxobutanoïque, les solvates, les énantiomères et les sels de ces acides.

9. Composition selon la revendication 8, **caractérisée en ce que** le composé de formule (I) est choisi parmi :
- l'acide (-) 2-benzyl-4-(4-méthoxyphényl)-4-oxobutanoïque
- l'acide (+) 2-benzyl-4-(4-méthoxyphényl)-4-oxobutanoïque
- l'acide (-) 2-benzyl-4-(4-fluorophényl)-4-oxobutanoïque
- l'acide (+) 2-benzyl-4-(4-fluorophényl)-4-oxobutanoïque

10. Composition selon l'une quelconque des revendications précédentes, appropriée à une administration par voie orale.

11. Utilisation de metformine éventuellement sous la forme de l'un de ses sels pharmaceutiquement acceptables en association avec un composé de formule (I) tel que défini dans la revendication 1, pour la préparation d'une association médicamenteuse destinée à traiter le diabète.

12. Utilisation de metformine éventuellement sous la forme de l'un de ses sels pharmaceutiquement acceptables en association avec un composé de formule (I) tel que défini dans la revendication 1, pour la préparation d'une association médicamenteuse destinée à traiter le diabète non insulino-dépendant.

13. Utilisation de metformine éventuellement sous la forme de l'un de ses sels pharmaceutiquement acceptables en association avec un composé de formule (I) tel que défini dans la revendication 1, pour la préparation d'une association médicamenteuse destinée à traiter au moins une des pathologies associées au syndrome d'insulinorésistance, plus particulièrement choisie parmi la dyslipidémie, l'obésité, l'hypertension artérielle, les complications micro- et macro-vasculaires, comme l'athérosclérose, les rétinopathies, les néphropathies et les neuropathies.

14. Utilisation selon l'une quelconque des revendications 11 à 13, **caractérisée en ce que** le sel de metformine est un chlorhydrate, un fumarate, un embonate ou un chlorophénoxyacétate.

15. Utilisation selon l'une des revendications 11 à 14, **caractérisée en ce que** le composé de formule (I) est choisi parmi :
l'acide 2-benzyl-4-(4-méthoxyphényl)-4-oxobutanoïque
l'acide 2-benzyl-4-(4-fluorophényl)-4-oxobutanoïque
l'acide 2-cyclohexylméthyl-4-(4-méthoxyphényl)-4-oxobutanoïque
l'acide 2-benzyl-4-phényl-4-oxobutanoïque
l'acide 2-(β-naphthylméthyl)-4-phényl-4-oxobutanoïque
l'acide 2-benzyl-4-(β-naphtyl)-4-oxobutanoïque
l'acide 2-[(4-chlorophényl)méthyl]-4-(4-méthoxyphényl)-4-oxobutanoïque
l'acide 2-benzyl-4-(4-méthylphényl)-4-oxobutanoïque
l'acide 4-(4-fluorophényl)-2-[(4-méthoxyphényl)méthyl]-4-oxobutanoïque
l'acide 2-benzyl-4-(3,4-méthylènedioxyphényl)-4-oxobutanoïque
l'acide 2-benzyl-4-cyclohexyl-4-oxobutanoïque
l'acide 4-phényl-2-[(tétrahydrofur-2-yl)méthyl]-4-oxobutanoïque,
les solvates, les énantiomères et les sels de ces acides.

16. Utilisation selon l'une des revendications 11 à 14, **caractérisée en ce que** le composé de formule (I) est choisi parmi :
- l'acide (-) 2-benzyl-4-(4-méthoxyphényl)-4-oxobutanoïque
- l'acide (+) 2-benzyl-4-(4-méthoxyphényl)-4-oxobutanoïque
- l'acide (-) 2-benzyl-4-(4-fluorophényl)-4-oxobutanoïque
- l'acide (+) 2-benzyl-4-(4-fluorophényl)-4-oxobutanoïque

17. Utilisation selon l'une quelconque des revendications 11 à 16, **caractérisée en ce que** l'association médicamenteuse se présente sous la forme d'une dose unitaire contenant la metformine ou l'un de ses sels pharmaceutiquement acceptables, et un composé de formule (I).

18. Utilisation selon la revendication 17, **caractérisée en ce que** la dose unitaire comprend de 50 à 1000 mg de metformine et de 12,5 à 400 mg de composé de formule (I).
